# EUROPÄISCHE PATENTSCHRIFT

(11) **EP 0 470 954 B1**
(45) Veröffentlichungstag und Bekanntmachung des Hinweises auf die Patenterteilung: **16.03.1994**
(21) Anmeldenummer: 89909159.9
(22) Anmeldetag: 23.08.1989
(51) Int. Cl.: A61B 8/12

(54) **VORRICHTUNG ZUR TRANSÖSOPHAGEALEN ECHOKARDIOGRAPHIE**
DEVICE FOR TRANSOESOPHAGAL ECHOCARDIOGRAPHY
DISPOSITIF D'ECHOCARDIOGRAPHIE TRANS SOPHAGIENNE

(30) Priorität: 03.05.1989 DE 3914619
(43) Veröffentlichungstag der Anmeldung: 19.02.1992
(73) Patentinhaber: TomTec Tomographic Technologies Inc., Northbrook, Illinois 60062 (US)
(72) Erfinder: WOLLSCHLÄGER, Helmut, D-7800 Freiburg (DE); WOLLSCHLÄGER, Susanna, D-7800 Freiburg (DE); ZEIHER, Andreas, D-7800 Freiburg (DE); KLEIN, Hans-Peter, D-8017 Ebersberg (DE)
(74) Vertreter: Rackette, Karl, Dipl.-Phys. Dr.-Ing
(86) Internationale Anmeldenummer: DE8900550
(87) Internationale Veröffentlichungsnummer: WO9013259

(56) Entgegenhaltungen:
- EP-A- 48 013
- EP-A- 234 951
- GB-A- 2 015 733
- GB-A- 2 156 997
- Ultrasound in Medicine and Biology, vol.12, no.12, Dezember 1986, Seiten 965-975; R.W.Martin et al: "An endoscopic micromanipulator for multiplanar transesophageal imaging"

## Beschreibung

Die Erfindung betrifft eine Vorrichtung zur transösophagealen Echokardiographie mit einer zur Erzeugung multiplanarer Schichtbilder des Herzens eines Patienten einen Ultraschallwandler aufweisenden flexiblen Ultraschallsonde, die über ein Steuer- und Auswertegerät an ein Bildverarbeitungssystem angeschlossen ist.

Eine derartige Vorrichtung ist aus Roy W. Martin et al, An Endoscopic Micromanipulator for Multiplanar Transesophageal Imaging, Ultrasound in Med. & Biol. Vol. 12, No. 12, pp. 965 - 975, 1986 bekannt und dient zur dreidimensionalen Rekonstruktion eines Ultraschallherzbildes, indem eine Vielzahl von Schnittbildern mit Hilfe eines Ultraschallwandlers aufgenommen wird, der am vorderen Ende eines Endoskopes angebracht ist und um eine quer zur Längsachse des Endoskopes verlaufende Achse verschwenkbar ist. Auf diese Weise wird eine Vielzahl von Schichtbildern erhalten, deren zugeordnete Schnittebenen schräg zueinander verlaufen. Dies führt dazu, daß die gewonnenen Bilddaten sowohl in geometrischer als auch in optischer Hinsicht eine geringe Qualität haben und das Abtastvolumen klein und ungünstig verteilt ist. Außerdem sind die Abtastungen nicht reproduzierbar, so daß es nicht möglich ist, die für Vergleichsstudien erforderliche Qualität dreidimensionaler Bilder zu erhalten.

In einem Aufsatz von Michael Schlüter et al., Transesophageal Two-Dimensional Echocardiography: Comparison of Ultrasonic and Anatomic Sections, The American Journal of Cardiology, Vol. 53, Seiten 1173-1178, 1984, ist eine Vorrichtung zur transösophagealen Echokardiographie beschrieben, die es gestattet, horizontale Schichtbilder zu erhalten. Dabei werden tomographische Schnitte durch das Herz dadurch erhalten, daß ein Gastroskop im Ösophagus verschoben und gedreht wird. Die Notwendigkeit der manuellen, nicht reproduzierbaren Bewegung macht es unmöglich, aus zweidimensionalen Schichtbildern dreidimensionale plastische Bilder zusammenzusetzen.

Aus der US-PS 4 327 738 sind ein endoskopisches Verfahren sowie eine Vorrichtung zur Durchführung einer Ultraschall-B-Bildabtastung bekannt. Der Ultraschallwandler ist im vorderen starren Endstück eines ansonsten flexiblen Schlauches angeordnet. Um multiplanare Tomogramme zu erzeugen, sind schwer reproduzierbare Lageveränderungen erforderlich, so daß exakte räumliche Rekonstruktionen der abgetasteten Organe nicht möglich sind.

Ausgehend von diesem Stand der Technik liegt der Erfindung die Aufgabe zugrunde, eine Vorrichtung zur transösophagialen Echokardiographie zu schaffen, die es gestattet, reproduzierbare topographische Informationen über das Herz und seine Bewegungen zu erhalten.

Diese Aufgabe wird erfindungsgemäß dadurch gelöst, daß der Ultraschallwandler zur Abtastung einer Folge parallel zueinander verlaufender Schichtebenen des Herzens auf einem in der Ultraschallsonde axial entlang einer Geraden schrittweise verfahrbaren Gleitschlitten angeordnet ist, daß ein Zwischenspeicher vorgesehen ist, in den bei der Abtastung jeder Schicht herzphasensynchron wenigstens ein Schichtbild abspeicherbar ist, daß ein Hauptspeicher für wenigstens ein aus einer Folge von parallelen Schichtbildern zusammengesetztes dreidimensionales Bild vorgesehen ist, dessen Dateneingang über eine Selektionsstufe mit einem Datenausgang des Zwischenspeichers verbunden ist, durch die das herzphasensynchrone Schichtbild oder die herzphasensynchronen Schichtbilder nur bei gleich lange dauernden Herzzyklen zum Hauptspeicher weiterleitbar sind, und daß ein Vorschubsignal zum Verfahren des Ultraschallwandlers in die nächste Schichtebene nur nach Übernahme des Zwischenspeicherinhaltes in den Hauptspeicher erzeugt wird.

Dadurch, daß der Ultraschallwandler entlang einer Geraden schrittweise verfahren wird, werden parallel zueinander verlaufende Schnittebenen und dementsprechend parallel zueinander ausgerichtete Schichtbilder erzeugt. Der Ultraschallwandler befindet sich auf einem Gleitschlitten, der im distalen Ende der Ultraschallendoskopeinrichtung, das eine Ösophagussonde darstellt, axial verschiebbar ist. Damit die Ösophagussonde für die transösophagiale Echokardiographie in den Ösophagus leicht eingeführt werden kann, ist diese flexibel ausgebildet, wobei jedoch eine Versteifung des distalen Endbereichs für eine präzise Orientierung der Abtastebenen vorgesehen ist. Dies wird durch eine Vielzahl von Führungsgliedern erreicht, die vor Beginn der Abtastung der Schichtebenen mit ihren Stirnseiten gegeneinander gezogen werden. Im versteiften Zustand bilden diese Führungsglieder einen geraden und stabilen Führungskanal, so daß der Gleitschlitten mit dem Ultraschallwandler entlang einer Geraden schrittweise verfahrbar ist.

Zum Verfahren des Gleitschlittens sowie zur wahlweisen Versteifung des distalen Endes der Ösophagussonde ist eine Manipulationseinrichtung vorgesehen, die einen Schrittmotor umfaßt, dessen Motorvorschub durch Auswertung eines Elektrokardiogramms synchronisiert ist.

Die Vorrichtung verfügt über einen Zwischenspeicher, in den vorzugsweise eine Vielzahl von Schichtbildern abgespeichert wird, die zur gleichen Schnittebene, jedoch zu verschiedenen Zeiten oder Phasen eines Herzzyklus gehören. Bei konstanter Herzfrequenz, Atemlage und räumlicher Ösophagussondenlage werden die im Zwischenspeicher gespeicherten Schichtbilder in einen Hauptspeicher übernommen, der es gestattet, die zu gleichen Herzphasen gehörenden Schichtbilder aufeinanderfolgender Herzzyklen zu dreidimensionalen Bilddatensätzen sortiert zu vereinen. Der Hauptspeicher enthält somit für jeden inkrementalen Schritt des Ultraschallwandlers in axialer Richtung des Ösophagus entsprechend der zeitlichen Auflösung eines Herzzyklus oder der Bildwiederholrate des Ultraschallgerätes eine Reihe von dreidimensionalen Rekonstruktionen des Herzens oder Datenwürfeln.

Damit nur solche Schichtbilder zu dreidimensionalen Bilddatensätzen zusammengesetzt werden, die bei konstanten Bedingungen aufgenommen worden sind, ist zwischen dem Zwischenspeicher und dem Hauptspeicher eine Selektionsstufe vorgesehen. Nur wenn der Inhalt des Zwischenspeichers den Kriterien für eine Weiterleitung an den Hauptspeicher genügt, erfolgt ein Vorschub des Gleitschlittens mit dem Ultraschallwandler durch den Schrittmotor und damit die Ansteuerung einer benachbarten Schnittebene.

Bei einem zweckmäßigen Ausführungsbeispiel der Erfindung ist eine Einrichtung zur Erfassung eines Elektrokardiogramms vorgesehen, die einerseits für die Synchronisation des Motorvorschubes vorgesehen ist. Andererseits gestattet sie die Synchronisierung oder Triggerung der in zeitlich versetztem Abstand während eines Herzzyklus pro Schnittebene gemachten Aufnahmen. Weiterhin dient das erfaßte Elektrokardiogramm zur Bestimmung des zeitlichen Abstandes aufeinanderfolgender R-Zacken im EKG und damit zur Erfassung sowie Überwachung einer konstanten Herzfrequenz, d.h. eines konstant lange dauernden Herzzyklus. Auf diese Weise wird sichergestellt, daß die in einem festen zeitlichen Raster aufeinanderfolgenden Schichtaufnahmen jeweils gleichen Phasen eines Herzzyklus zugeordnet sind. Würde beispielsweise der Herzzyklus infolge eines schneller schlagenden Herzens kürzer, würde das n-te-Schichtbild nicht mehr der ursprünglich zugeordneten Herzphase, sondern einer späteren Herzphase zugeordnet, so daß ein dreidimensionaler Bilddatensatz Schichtbilder enthalten würde, die bei verschiedenen Herzphasen aufgenommen worden sind. Ein derartiger Bilddatensatz ist jedoch wegen der Bewegung des Herzens nicht brauchbar.

Zweckmäßige Ausgestaltungen und Weiterbildungen der Erfindung sind Gegenstand von Unteransprüchen.

Nachfolgend wird die Erfindung anhand eines Ausführungsbeispiels näher beschrieben. Es zeigen:
- Fig. 1: eine schematische Darstellung der erfindungsgemäßen Vorrichtung zur transösophagealen Echokardiographie,
- Fig. 2: eine perspektivische Ansicht des Ösophaguskopes oder distalen Endes der Endoskopieeinrichtung in zwei Positionen in der Nähe des zu überwachenden Herzens,
- Fig. 3: eine schematische und bildliche Veranschaulichung der der Erfindung zugrunde liegenden Steuerlogik und
- Fig. 4: eine schematische Darstellung zur Veranschaulichung der Erzeugung einer beliebigen Schnittebene durch ein dreidimensionales Bild eines Herzens.

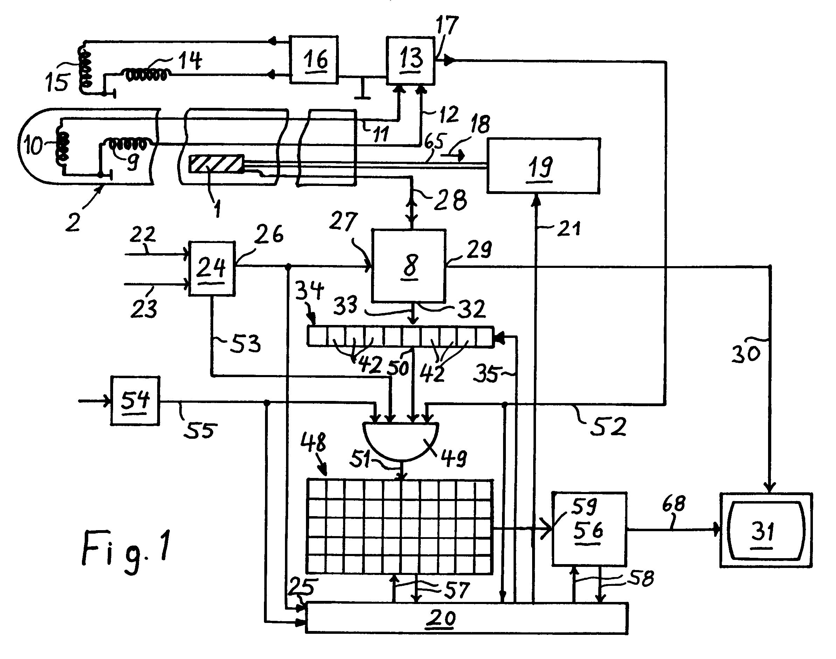

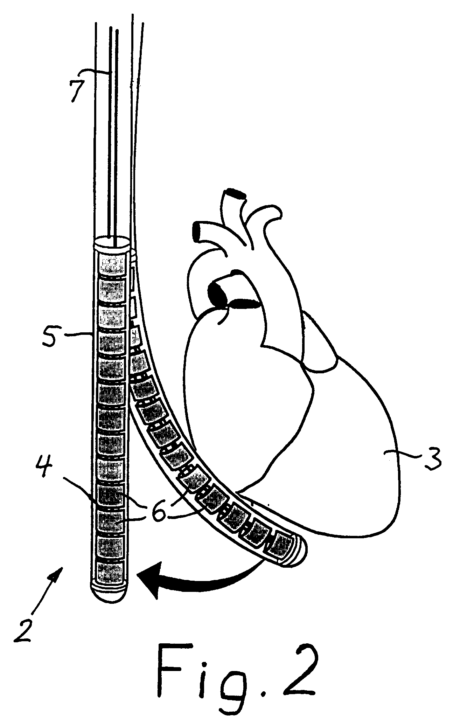

Die in Fig. 1 schematisch dargestellte Vorrichtung zur transösophagealen Echokardiographie verfügt über einen Ultraschallkopf 1, beispielsweise in Gestalt eines mechanischen Sektorscanners oder Phased Array, der in axialer Richtung entlang dem vorderen Ende einer Ösophagussonde 2 verschiebbar ist, die in Fig. 2 in vereinfachter Weise in zwei unterschiedlichen Lagen zusammen mit einem Herz 3 dargestellt ist. Die Ösophagussonde 2 verfügt über einen distalen Endbereich 4 mit einem flexiblen Schlauch 5, durch den eine Vielzahl von starren Führungsgliedern 6 zusammengehalten werden. Aufgrund der Elastizität des flexiblen Schlauches 5 ist der distale Endbereich 4 leicht verbiegbar, was in Fig. 2 durch den nach rechts gekrümmten Teil der Ösophagussonde 2 veranschaulicht ist.

Im Innern der Führungsglieder 6 ist ein Führungskanal vorgesehen, in dem ein in der Zeichnung nicht dargestellter Gleitschlitten mit dem Ultraschallkopf 1 in axialer Richtung längsverschieblich bewegbar ist. Um eine Streckung und Versteifung des distalen Endbereichs 4 für eine präzise Orientierung der Abtastebenen des Ultraschallkopfes 1 zu gewährleisten, sind in den Führungsgliedern 6 Bohrungen vorgesehen, durch die sich ein Spanndraht 7 erstreckt, der mit Hilfe eines Bowdenzuges spannbar ist, so daß die einzelnen Führungsglieder 6 fest gegeneinander gepreßt werden und eine präzise Führung des Gleitschlittens für den Ultraschallkopf 1 gestatten. Beim Zusammenziehen der Führungsglieder 6 mit Hilfe des Spanndrahtes 7 liegen diese in der in Fig. 2 links gezeichneten Weise mit ihren Stirnseiten fest gegeneinander an und führen somit zu einer Versteifung und Ausrichtung des distalen Endbereichs 4 entlang einer Geraden. Nach dem Ausrichten des distalen Endbereichs 4 in der in Fig. 2 links gezeigten Art und Weise werden mit Hilfe des Ultraschallkopfes 1 und des angeschlossenen Ultraschalldiagnostikgerätes 8 eine Vielzahl von Schichtbildern entsprechend den durch die Lage der Ösophagussonde 2 und die Position des Ultraschallkopfes 1 definierten Schnittebenen aufgenommen. Dies erfolgt, sobald der Patient sich an die eingeführte Ösophagussonde 2 angepaßt hat und seine Herzfrequenz, seine Atemlage und die Lage der Ösophagussonde 2 stabilisiert sind.

Da die Lage der Schnittebenen durch das Herz 3 abhängig von der räumlichen Sondenlage der Ösophagussonde 2 sind, was sich aus Fig. 2 unmittelbar ergibt, ist zu Gewährleistung der Parallelität aller Schnittebenen, für die Schichtbilder aufgenommen und gespeichert werden, eine Detektion der räumlichen Sondenlage vorgesehen. Zu diesem Zweck verfügt die Ösophagussonde 2 an einem ihrer Führungsglieder 6 oder an ihrem vorderen Ende über eine Längsspule 9 und eine Querspule 10, die über Leitungen 11 und 12 mit einer Lageerfassungseinrichtung 13 verbunden sind.

Die Längsspule 9 und die Querspule 10 befinden sich im Feld zweier orthogonal ausgerichteten Induktionsschleifen 14, 15, die über einen Taktgenerator 16 zur Erzeugung eines festen Koordinatensystems alternierend getaktet werden und je nach der räumlichen Lage der Ösophagussonde 2 mit der Längsspule 9 sowie der Querspule 10 in diesen unterschiedliche Spannungen induzieren, die in der Lageerfassungseinrichtung 13 ausgewertet werden.

Die Lageerfassungseinrichtung 13 gestattet nicht nur die Bestimmung der jeweiligen augenblicklichen Lage der Ösophagussonde 2, sondern auch eine laufende Lageerfassung sowie eine Häufigkeitsanalyse bezüglich der verschiedenen Orientierungen und Lagen, so daß automatisch eine ausgezeichnete Lage oder standardisierte Richtung ermittelt werden kann, sobald der Patient beruhigt ist und mit der Aufnahme der Schichtbilder begonnen werden soll.

Am Ausgang 17 der Lageerfassungseinrichtung 13 tritt jedesmal dann ein Freigabesignal auf, wenn die gewünschte häufigste räumliche Sondenlage der Ösophagussonde 2 vorliegt. Bei dieser am häufigsten oder normalerweise auftretenden Sondenlage kann damit gerechnet werden, daß der größte Anteil der erfaßten Schichtbilder aus parallel zueinander verlaufenden Schnittebenen herrührt. Die Induktionsschleifen 14 und 15 sind in der Liege für den Patienten integriert, wobei eine der Induktionsschleifen parallel zur Liegefläche der Patienten liegt und die andere in einer Seitenwand rechtwinklig zur Liegefläche integriert ist. Die beiden Induktionsschleifen 14 und 15 definieren ein räumlich festes Koordinatensystem, das als Bezugspunkt für die Lage der mit Hilfe des Ultraschallkopfes 1 abgetasteten Schnittebene dient.

Jedesmal wenn eine Schnittebene oder Schicht des Herzens abgetastet worden ist und für eine vorgegebene Schicht oder Schnittebene mehrere zu verschiedenen Phasen eines Herzzyklus gehörende Schichtbilder erzeugt worden sind, wird der Ultraschallkopf 1 in Richtung eines Pfeils 18 mit Hilfe eines Schrittmotors 19 verfahren. Der Schrittmotor 19 ist dazu über einen Bowdenzug 65 mit dem den Ultraschallkopf 1 tragenden Schlitten verbunden und gestattet es beispielsweise, nach jedem Herzzyklus den Ultraschallkopf 1 in axialer Richtung um 0,5 mm zurückzuziehen. Bei einem Verfahrweg von 10,5 cm ergeben sich somit 210 Schnittebenen, die rechtwinklig zur Längsachse der Ösophagussonde 2 liegen und sich durch das benachbarte Herz 3 erstrecken. Der Motorvorschub des Schrittmotors 19 ist EKG-synchronisiert. Die aus dem EKG des Patienten abgeleiteten Synchronisiersignale werden mit Hilfe eines Rechners 20 erzeugt und über eine Synchronisierleitung 21 zum Schrittmotor 19 und dessen Steuerelektronik geführt.

Das EKG des Patienten wird mit Hilfe von Elektroden 22, 23 erfaßt, die mit einem EKG-Gerät 24 verbunden sind. Das EKG-Gerät 24 ist mit einem EKG-Eingang 25 des Rechners 20 verbunden, der auch die erforderlichen Steuerungsaufgaben für die Komponenten der in Fig. 1 gezeigten Vorrichtung übernimmt. Der erste Ausgang 26 des EKG-Gerätes 24 ist weiterhin mit einem Steuereingang 27 des Ultraschalldiagnosegerätes 8 verbunden, um Schichtbildaufnahmen durch Triggerung über die R-Zacken im Elektrokardiogramm zu steuern.

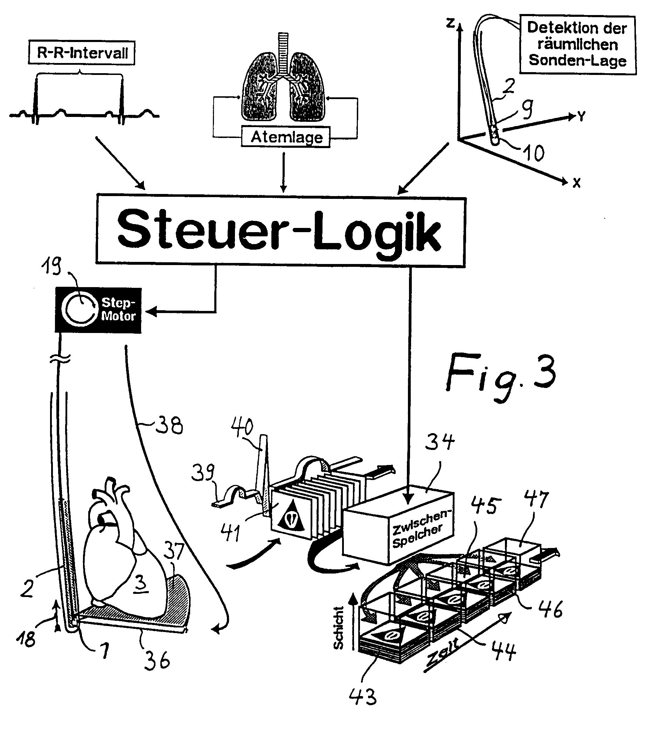

Sobald dem Steuereingang 27 ein Triggerimpuls zugeführt wird, beginnt der Ultraschallkopf 1 entsprechend der Bildwiederholrate des Ultraschalldiagnostikgerätes 8 die der jeweiligen Lage und Orientierung des Ultraschallkopfes 1 zugeordnete Schnittebene abzutasten, um im zeitlichen Abstand, d.h. zu verschiedenen Herzphasen innerhalb eines Herzzyklus Schichtbilder 41 (Fig. 3) zu erfassen. Dazu ist der Ultraschallkopf 1 über eine Signalleitung 28 mit dem Ultraschalldiagnostikgerät 8 verbunden. Am ersten Bildausgang 29 des Ultraschalldiagnostikgerätes 8 liegen die einzelnen Schichtbilder 41 als Signal an und können über eine Videoleitung 30 zum Monitor 31 geführt werden, der eine laufende Beobachtung der zeitlich schnell aufeinanderfolgenden Schichtbilder 41 gestattet. Je nach dem Ultraschalldiagnostikgerät 8 dauert die Erfassung eines Schichtbildes 13 msec, wobei die Schichtbilder 41 im Intervall von etwa 33 msec aufeinanderfolgen. Bei einer Herzzyklusdauer von 0,8 sec können somit etwa zwei Dutzend Schichtbilder 41 pro Herzzyklus aufgenommen werden.

Der zweite Bildausgang 32 des Ultraschalldiagnostikgerätes 8 ist über eine Datenleitung 33 mit dem Dateneingang eines Zwischenspeichers 34 verbunden. Über eine Adressleitung 35 steuert der Rechner 20 die Speicherplätze des Zwischenspeichers 34 so an, daß die sequentiell zu verschiedenen Zeitpunkten innerhalb eines Herzzyklus erzeugten Schichtbilder 41 im Zwischenspeicher 34 unabhängig von der jeweiligen Herzfrequenz des Patienten oder dessen Atemlage und der räumlichen Sondenlage gespeichert werden.

Fig. 3 veranschaulicht in einer perspektivischen Ansicht die Funktionsweise der in Fig. 1 blockschaltbildartig dargestellten Vorrichtungen zur transösophagealen Elektrokardiographie. Man erkennt neben dem Herzen 3 die Ösophagussonde 2 und den in Richtung des Pfeils 18 verfahrbaren Ultraschallkopf 1, der es gestattet, parallel zueinander verlaufende sektorförmige Schnittebenen 36 und 37 abzutasten. Ein Pfeil 38 verweist auf den Abstand zwischen den Schnittebenen 36 und 37 nach dem Betätigen des Schrittmotors 19, der über einen Bowdenzug mit dem den Ultraschallkopf 1 tragenden Gleitschlitten verbunden ist.

Aufgrund der im Rechner 20 gespeicherten Steuerlogik arbeiten die in Fig. 1 dargestellten Komponenten der Vorrichtung zur transösophagealen Echokardiographie in der in Fig. 3 veranschaulichten Weise zusammen.

In Fig. 3 erkennt man den Wellenzug 39 eines Elektrokardiogramms und insbesondere die zu Beginn eines Herzzyklus auftretende R-Zacke 40. Während des Herzzyklus werden etwa alle 30 oder alle 33 msec. mit Hilfe des Ultraschallkopfes 1 und des Ultraschalldiagnostikgerätes 8 Schichtbilder 41 aufgenommen, die in Fig. 3 der Schnittebene 37 zu verschiedenen Zeitpunkten oder Herzphasen eines Herzzyklus zugeordnet sind. Die in Fig. 3 schematisch dargestellten Schichtbilder 41, deren Zahl zwischen 1 und mehreren Dutzend liegen kann, werden unter der Steuerung des Rechners 20 in den Zwischenspeicher 34 übernommen, wobei die Adressleitung 35 sequentiell einzelnen Schichtbildern 41 zugeordnete Speicherbereiche 42 freigibt.

Da die Schichtbilder 41 einer Schnittebene, insbesondere der Schnittebene 37 zu verschiedenen Zeitpunkten oder Phasen eines Herzzyklus aufgenommen worden sind, werden diese in der in Fig. 3 veranschaulichten Weise auf Datenwürfel 43 bis 47 verteilt, die mit Hilfe eines Hauptspeichers 48 realisiert werden. Fig. 3 veranschaulicht, wie die zu verschiedenen Zeitpunkten eines Herzzyklus aber zu einer gleichen einzigen Schnittebene gehörenden Schichtbilder 41 vom Zwischenspeicher 34 auf die Datenwürfel 43 bis 47 des Hauptspeichers 48 verteilt werden. Man erkennt, daß nach dem Abtasten des gesamten Herzvolumens die Datenwürfel 43 bis 47 vollständige dreidimensionale plastische Darstellungen enthalten, die durch Zusammenfügen von Schichtbildern 41 erzeugt worden sind. Jedem Datenwürfel 43 bis 47 ist dabei innerhalb eines Herzzyklus eine andere Herzphase zugeordnet. Obwohl das Ultraschalldiagnostikgerät 8 es nicht gestattet, das gesamte Volumen des Herzens 3 innerhalb einer gegenüber der Herzbewegung vernachlässigbaren Zeit ganz abzutasten, enthalten die Datenwürfel 43 bis 47 jeweils ein räumliches Standbild des gesamten Herzens.

Bei der vorliegenden Betrachtung wurde jedoch vorausgesetzt, daß während einer Anzahl von Herzzyklen, die mit der Zahl der Schichtbilder 41 in einem der Datenwürfel 43 bis 47 übereinstimmt, keine Veränderungen bezüglich der Orientierung der Ösophagussonde 2, der Herzfrequenz und der Atemlage des Patienten erfolgten. Da dies in der Praxis nicht erreichbar ist, ist die in Fig. 3 schematisch dargestellte Steuerlogik mit der in Fig. 1 dargestellten Selektionsstufe 49 vorgesehen.

Die Selektionsstufe 49 verbindet den Ausgang 50 des Zwischenspeichers 34 mit dem Dateneingang 51 des Hauptspeichers 48. Bei durchgeschalteter Selektionsstufe 49 werden die in den Speicherbereichen 42 abgelegten Schichtbilder 41 sequentiell in die schematisch dargestellten Datenwürfel 43 bis 47 übernommen. Fehlt jedoch beispielsweise am Ausgang 17 der Lageerfassungseinrichtung 13 das Freigabesignal, so wird die Selektionsstufe 49 über die Freigabeleitung 52 gesperrt und mit Hilfe des Ultraschalldiagnostikgerätes 8 eine neue Folge von Schichtbildern 41 erzeugt und im Zwischenspeicher 34 abgelegt. Der Zwischenspeicher 34 wird so lange mit einem neuen Satz von Schichtbildern 41 überschrieben, bis die Selektionsstufe 49 ein Durchschalten zum Hauptspeicher 48 gestattet.

Das EKG-Gerät 24 ist ebenfalls über eine Freigabeleitung 53 mit einem zugeordneten Eingang der Selektionsstufe 49 verbunden. Die Freigabeleitung 53 ist nur dann aktiv, wenn im EKG-Gerät 24 durch Auswerten des R-R-Intervalles festgestellt worden ist, daß dieses Intervall innerhalb eines Toleranzbereichs von ± 5 % konstant geblieben ist. Das EKG-Gerät 24 ermittelt selbstätig, bei welcher Intervallänge eine Freigabe erfolgen soll. Diese ist insbesondere dann gegeben, wenn der Patient etwa 1 Minute nach dem Einführen der Ösophagussonde mit einer stabilisierten mittleren Herzfrequenz gleichmäßig atmet.

Ein Atmungsdetektor 54, der mit dem EKG-Gerät 24 gekoppelt sein kann, überwacht die Atmungskurve des Patienten und erzeugt beim Erreichen einer konstanten Atemlage auf einer weiteren Freigabeleitung 55 ein Freigabesignal für die Selektionsstufe 49.

Aus den obigen Ausführungen erkennt man, daß nur dann, wenn das R-R-Intervall, die Atemlage und die räumliche Sondenlage konstant sind, eine Übernahme der Daten aus dem Zwischenspeicher 34 in den Hauptspeicher 48 erfolgt.

Aus Fig. 1 ergibt sich weiterhin, daß der Rechner 20 mit den Ausgangssignalen der Lageerfassungseinrichtung 13, des EKG-Gerätes 24 und des Atmungsdetektors 54 beaufschlagt ist. Unter Auswerten der verschiedenen Eingangssignale steuert der Rechner 20 den Funktionsablauf der in Fig. 1 dargestellten Vorrichtung zur transösophagealen Echokardiographie.

Dem Hauptspeicher 48 und dem Rechner 20 ist in der in Fig. 1 dargestellten Weise ein digitales Bildverarbeitungssystem 56 zugeordnet. Der Rechner 20 steht über Steuerleitungen 57 und 58 mit dem Hauptspeicher 48 und dem Bildverarbeitungssystem 56 in Verbindung. Der Ausgang des Hauptspeichers 58 ist mit dem Eingang 59 des Bildverarbeitungssystems verbunden.

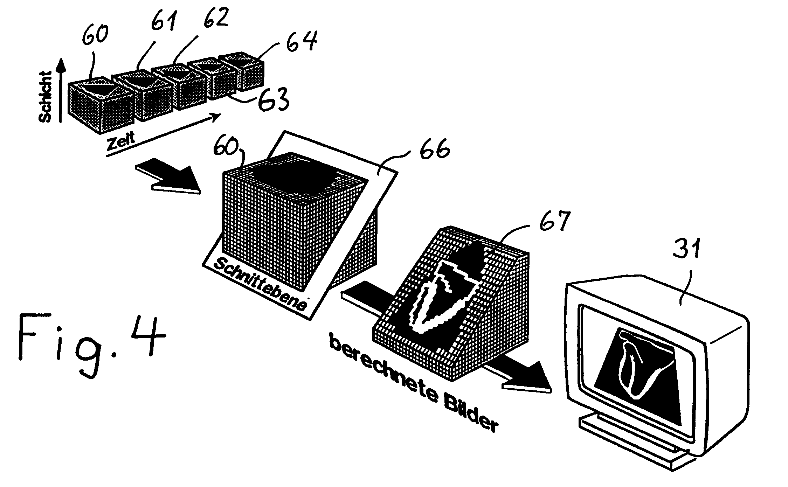

In Fig. 4 sind die im Hauptspeicher 48 gespeicherten herzphasensynchronen dreidimensionalen Bilder 60 bis 64 als Datenwürfel aus einer Vielzahl von Voxel dargestellt. Die dreidimensionalen Bilder 60, 61, 62, 63, 64 bestehen aus isotropischen kubischen Datensätzen, wobei jede Bildschicht, beispielsweise aus 256 x 256 Pixel zusammengesetzt sein kann. Zur Darstellung der Grauwerte der Bildpunkte oder Voxel können beispielsweise jeweils 8 Bit verwendet werden.

Wenn beispielsweise das dreidimensionale Bild 60 in das Bildverarbeitungssystem 56 übernommen wird, so gestattet es das Bildverarbeitungssystem 56 durch den in Fig. 4 als Würfel dargestellten Datensatz eine beliebige Schnittebene 66 zu legen und das zugeordnete neue Schnittbild 67 zu berechnen. Die Orientierung der zugeordneten Schnittebene 66 stimmt dabei nicht notwendigerweise mit der relativen Lage einer der Schnittebenen 36 oder 37 überein. Das berechnete Schnittbild 67 kann dann über eine Ausgangsleitung 68 des Bildverarbeitungssystems 56 zum Monitor 31 geführt und auf dessen Bildschirm in der in Fig. 4 veranschaulichten Weise dargestellt werden.

## Patentansprüche

1. Vorrichtung zur transösophagealen Echokardiographie mit einer zur Erzeugung multiplanarer Schichtbilder des Herzens (3) eines Patienten einen Ultraschallwandler (1) aufweisenden flexiblen Ultraschallsonde (2), die über ein Steuer- und Auswertegerät (8) an ein Bildverarbeitungssystem (56) angeschlossen ist, **dadurch gekennzeichnet,**
daß der Ultraschallwandler (1) zur Abtastung einer Folge von parallel zueinander verlaufenden Schichtebenen (36, 37) des Herzens (3) auf einem in der Ultraschallsonde (2) axial entlang einer Geraden schrittweise verfahrbaren Gleitschlitten angeordnet ist, daß ein Zwischenspeicher (34) vorgesehen ist, in den bei der Abtastung jeder Schicht (36, 37) herzphasensynchron wenigstens ein Schichtbild (41) abspeicherbar ist, daß ein Hauptspeicher (48) für wenigstens ein aus einer Folge von parallelen Schichtbildern (41) zusammengesetztes dreidimensionales Bild (43 - 47, 60 - 64) vorgesehen ist, dessen Dateneingang (51) über eine Selektionsstufe (49) mit einem Datenausgang (50) des Zwischenspeichers (34) verbunden ist, durch die das herzphasensynchrone Schichtbild oder die herzphasensynchronen Schichtbilder (41) nur bei gleich lange dauernden Herzzyklen zum Hauptspeicher (48) weiterleitbar sind, und daß ein Vorschubsignal (21) zum Verfahren des Ultraschallwandlers (1) in die nächste Schichtebene (36, 37) nur nach Übernahme des Zwischenspeicherinhaltes (42) in den Hauptspeicher (48) erzeugt wird.

2. Vorrichtung nach Anspruch 1, dadurch gekennzeichnet, daß eine Einrichtung (24) zur Erfassung eines EKG vorgesehen ist, durch die das schrittweise Verfahren des Ultraschallwandlers (1) gesteuert ist.

3. Vorrichtung nach Anspruch 2, dadurch gekennzeichnet, daß die Einrichtung (24) zur Erfassung eines EKG eine Meßuhr zur Bestimmung des R-R-Intervalls aufeinanderfolgender EKG-Zyklen sowie der Häufigkeitsverteilung der Dauern der R-R-Intervalle aufweist und daß die Einrichtung (24) nur dann ein Freigabesignal (53) an die Selektionsstufe (49) leitet, wenn der Inhalt (41) des Zwischenspeichers (34) einem vorgegebenen konstanten R-R-Intervall zugeordnet ist.

4. Vorrichtung nach Anspruch 3, dadurch gekennzeichnet, daß bei jeder Schichtebenenposition des Ultraschallwandlers (1) herzphasensynchron eine Vielzahl von den verschiedenen Herzphasen eines Herzzyklus zugeordnete Schichtbilder (41) erzeugbar und im Zwischenspeicher (34) speicherbar sind, durch die nach der selektiven Übernahme in den Hauptspeicher (48) eine zeitliche Folge dreidimensionaler Bilder (60 bis 64) zu aufeinanderfolgenden Phasen eines Herzzyklus gebildet wird.

5. Vorrichtung nach einem der vorstehenden Ansprüche, dadurch gekennzeichnet, daß eine Lageerfassungseinrichtung (9, 10 und 13 bis 16) vorgesehen ist, durch die die räumliche Lage des den Gleitschlitten enthaltenden distalen Endbereichs (4) des Ultraschallendoskopes (2) erfaßbar ist.

6. Vorrichtung nach Anspruch 5, dadurch gekennzeichnet, daß die Lageerfassungseinrichtung (13) das Erfassen einer Häufigkeitsverteilung der verschiedenen Lagen gestattet, und daß die Selektionsstufe (49) nur dann mit einem Freigabesignal (17, 52) beaufschlagbar ist, wenn der Inhalt des Zwischenspeichers (34) einer vorgegebenen konstanten räumlichen Lage des distalen Endes (4) des Ultraschallendoskopes (2) zugeordnet ist.

7. Vorrichtung nach Anspruch 5 oder 6, dadurch gekennzeichnet, daß die Lageerfassungseinrichtung (13) zwei im distalen Ende (4) des Ultraschallendoskopes (2) vorgesehene rechtwinklig zueinander orientierte Spulen (9, 10) umfaßt, die mit einem Magnetfeld gekoppelt sind, das durch zwei alternierend getaktete orthogonal ausgerichtete Induktionsschleifen (14, 15) erzeugbar ist, von denen die eine in die Liegefläche einer Patientenliege und die andere in eine rechtwinklig neben der Liegefläche verlaufende Seitenwand zur Erzeugung eines festen Koordinatensystems integriert ist.

8. Vorrichtung nach einem der vorstehenden Ansprüche, dadurch gekennzeichnet, daß eine Einrichtung (54) zur Überwachung der Atemlage des Patienten vorgesehen ist, die ebenfalls mit der Selektionsstufe (49) verbunden ist, so daß eine Übernahme des Inhaltes des Zwischenspeichers (34) in den Hauptspeicher (48) und ein Inkrementieren der Lage der Schichtebenen (36, 37) um eine Schicht nur bei konstanter Atemlage (55) erfolgt.

9. Vorrichtung nach einem der vorstehenden Ansprüche, dadurch gekennzeichnet, daß dem Hauptspeicher (48) ein Rechner (20) zugeordnet ist, durch den aus den dreidimensionalen Bildern (60 bis 64) sich entlang beliebiger Schnittebenen (66) erstreckende Schnittbilder (67) errechenbar und auf den Monitor (31) der Bildverarbeitungseinrichtung (56) anzeigbar sind.

10. Vorrichtung nach Anspruch 9, dadurch gekennzeichnet, daß durch den Rechner (20) Herzvermessungen, insbesondere Bestimmungen der Kammervolumina, der Herzwanddicke, der Herzmuskelmassen und deren Veränderungen bei Vergleichsuntersuchungen, sowie Wandbewegungen während des Herzzyklus erfaßbar sind.

## Claims

1. A device for transoesophagal echocardiography comprising a flexible ultrasound probe (2) which has an ultrasound transducer (1) for producing multi-planar layer images of the heart (3) of a patient and which is connected by way of a control and evaluation device (8) to an image processing system (56) characterised in that the ultrasound transducer (11) for scanning a sequence of layer planes (36, 37) of the heart (3), which extend parallel to each other, is arranged on a slide carriage displaceable stepwise axially along a straight line, that there is provided an intermediate store (34) in which at least one layer image (41) can be stored in the scanning of each layer (36, 37) synchronously with the heart phase, that there is provided a main store (48) for at least one three-dimensional image (43-47, 60-64) composed of a sequence of parallel layer images (41), whose data input (51) is connected to a data output (50) of the intermediate store (34) by way of a selection stage (49), by which the layer image or images (41) which is or are synchronous with the heart phase can be transmitted to the main store (48) only in heart cycles of the same length, and that an advance signal (21) for moving the ultrasound transducer (1) into the next layer plane (36, 37) is produced only after transfer of the content of the intermediate store (42) into the main store (48).

2. A device according to claim 1 characterised in that there is provided a means (24) for detecting an ECG, by which the stepwise movement of the ultrasound transducer (1) is controlled.

3. A device according to claim 2 characterised in that the means (24) for detecting an ECG has a measuring clock for determining the R-R interval of successive ECG-cycles and the frequency distribution of the durations of the R-R intervals, and that the means (24) passes a release signal (53) to the selection stage (49) only when the content (41) of the intermediate store (34) is associated with a predetermined constant R-R interval.

4. A device according to claim 3 characterised in that in each layer plane position of the ultrasound transducer (1) a plurality of layer images (41) associated with the various heart phases of a heart cycle can be produced synchronously with the heart phases and stored in the intermediate store (34), by which after selective transfer into the main store (48) a temporal sequence of three-dimensional images (60 to 64) is formed for successive phases of a heart cycle.

5. A device according to one of the preceding claims characterised in that there is provided a position detection means (9, 10 and 13 to 16) by which the spatial position of the distal end region (4) of the ultrasound endoscope, which distal end region includes the slide carriage, can be detected.

6. A device according to claim 5 characterised in that the position detection means (13) permits detection of a frequency distribution of the various positions, and that the selection stage (49) can receive a release signal (17, 52) only when the content of the intermediate store (34) is associated with a predetermined constant spatial position of the distal end (4) of the ultrasound endoscope (2).

7. A device according to claim 5 or claim 6 characterised in that the position detection means (13) includes two coils (9, 10) which are oriented at a right angle to each other and which are disposed in the distal end (4) of the ultrasound endoscope (2) and which are coupled with a magnetic field which can be generated by two alternately cyclically actuated orthogonally oriented induction loops (14, 15) of which one is integrated into the couch surface of a patient couch and the other is integrated into a side wall extending at a right angle beside the couch surface to produce a fixed coordinate system.

8. A device according to one of the preceding claims characterised in that there is provided a means (54) for monitoring the respiratory state of the patient, which is also connected to the selection stage (49), so that transfer of the content of the intermediate store (34) into the main store (48) and incrementation of the position of the layer planes (36, 37) by one layer occurs only in a constant respiratory state (55).

9. A device according to one of the preceding claims characterised in that associated with the main store (48) is a computer (20) by which section images (67) extending along any section planes (66) can be calculated from the three-dimensional images (60 to 64) and displayed on the monitor (31) of the image processing device (56).

10. A device according to claim 9 characterised in that heart measurements, in particular determination of the chamber volumes, heart wall thickness, heart muscle masses and variations therein in comparative investigations as well as wall movements during the heart cycle can be detected by the computer (20).

## Revendications

1. Dispositif d'échocardiographie transoesophagienne comportant, pour obtenir des tomographies dans plusieurs plans du coeur (3) d'un patient, une sonde à ultra-sons flexible (2) munie d'un convertisseur à ultra-sons (1), et qui est raccordée, par l'intermédiaire d'un appareil de commande et d'analyse (8), à un système de traitement de l'image (56), caractérisé en ce que le convertisseur à ultra-sons (1) servant à l'exploration d'une succession de plans de coupe parallèles entre eux (36, 37) du coeur (3) peut être disposé sur un coulisseau susceptible de se déplacer axialement pas-à-pas le long d'une droite à l'intérieur de la sonde à ultra-sons (2), en ce qu'il est prévu une mémoire de stockage intermédiaire (34) dans laquelle, lors de l'exploration de chaque plan (36, 37) en synchronisme avec le cycle cardiaque, une tomographie (41) au moins peut être mémorisée, en ce qu'une mémoire principale (48), prévue pour au moins une image tridimensionnelle (43 - 47, 60 - 64) reconstituée à partir d'une séquence de tomographies parallèles (41), a son entrée de données (51) reliée à travers un étage de sélection (49) à une sortie de données (50) de la mémoire de stockage intermédiaire (34), par laquelle la tomographie synchrone avec le cycle cardiaque ou les vues en coupe (41) synchrones avec le cycle cardiaque ne peuvent être transmises à la mémoire principale (48) que si les cycles cardiaques ont constamment la même durée, et en ce qu'un signal d'avance (21) autorisant le déplacement du convertisseur à ultra-sons (1) vers le plan de coupe (36, 37) suivant ne peut être généré qu'après transfert du contenu (42) de la mémoire auxiliaire dans la mémoire principale (48).

2. Dispositif selon la revendication 1, caractérisé en ce qu'un dispositif (24) est prévu pour enregistrer un ECG, à l'aide duquel le déplacement pas à pas du convertisseur à ultra-sons (1) est piloté.

3. Dispositif selon la revendication 2, caractérisé en ce que le dispositif (24) prévu pour enregistrer un ECG comporte un comparateur pour déterminer l'intervalle R-R des cycles successifs de l'ECG ainsi que la distribution de fréquence des durées d'intervalle R-R, et en ce que le dispositif (24) ne délivre un signal de validation (53) à l'étage de sélection (49) que si le contenu (42) de la mémoire de stockage intermédiaire (34) correspond à un intervalle R-R constant prédéterminé.

4. Dispositif selon la revendication 3, caractérisé en ce que, à chaque position de plan de coupe du convertisseur à ultra-sons (1) un grand nombre de tomographies (41) correspondant à des phases différentes d'un cycle cardiaque sont obtenues en synchronisme avec les phases cardiaques et peuvent être enregistrées dans la mémoire de stockage intermédiaire (34), et à partir desquelles, après un transfert sélectif dans la mémoire principale (48) peut être constituée une suite chronologique d'images tridimensionnelles (60 à 64) correspondant aux phases successives d'un cycle cardiaque.

5. Dispositif selon l'une ou l'autre des revendications précédentes, caractérisé en ce qu'il est prévu un dispositif de détection de position (9, 10, et 13 à 16) permettant de déterminer la position spatiale de la zone d'extrémité distale (4) dans laquelle se trouve le coulisseau portant l'endoscope à ultra-sons (2).

6. Dispositif selon la revendication 5, caractérisé en ce que le dispositif de détection de position (13) permet de déterminer une distribution de fréquence des différentes positions, et que l'étage de sélection (49) ne peut recevoir un signal de validation (17, 52) que si le contenu de la mémoire de stockage intermédiaire (34) correspond à une position spatiale constante prédéterminée de l'extrémité distale (4) de l'endoscope à ultra-sons (2).

7. Dispositif selon l'une ou l'autre des revendications 5 et 6, caractérisé en ce que le dispositif de détection de position (13) comporte, dans l'extrémité distale (4) de l'endoscope à ultrasons (2), deux bobines (9, 10) orientées perpendiculairement l'une par rapport à l'autre et soumises à l'action d'un champ magnétique produit par deux boucles inductives (14, 15) disposées orthogonalement et commandées par des impulsions alternées, l'une d'entre elles étant disposée à la surface du lit du patient et l'autre dans une partie latérale perpendiculaire à la surface du lit, pour définir un système fixe de coordonnées.

8. Dispositif selon l'une ou l'autre des revendications précédentes, caractérisé en ce qu'un dispositif (54) prévu pour surveiller le rythme respiratoire du patient est également relié à l'étage de sélection (49), de telle sorte qu'un transfert du contenu de la mémoire de stockage intermédiaire (34) dans la mémoire principale (48), et une incrémentation d'un plan de la position des plans de coupe (36, 37) ne sont possibles que si le rythme respiratoire (55) est constant.

9. Dispositif selon l'une ou l'autre des revendications précédentes, caractérisé en ce qu'à la mémoire principale (48) est associé un calculateur (20) grâce auquel, à partir des images tridimensionnelles (60 à 64), il est possible de calculer des tomographies (67) s'étendant dans des plans de coupe (66) quelconques, et pouvant être vues sur l'écran du terminal (31) du système de traitement de l'image (56).

10. Dispositif selon la revendication 9, caractérisé en ce que, à l'aide du calculateur (20), des mesures des caractéristiques cardiaques peuvent être prises, en particulier en ce qui concerne le volume ventriculaire, l'épaisseur des parois, les masses musculaires et leurs modifications, par des études comparatives, ainsi que le mouvement des parois au cours du cycle cardiaque.
